(19) Europäisches Patentamt

European Patent Office

Office européen des brevets

(11) Publication number: **0 537 389 A1**

# EUROPEAN PATENT APPLICATION

(21) Application number: **91309531.1**

(22) Date of filing: **16.10.91**

(51) Int. Cl.5: **C07C 2/66**, C07C 2/86, C07C 6/12, C07C 15/085

(43) Date of publication of application: **21.04.93 Bulletin 93/16**

(84) Designated Contracting States: **BE DE FR GB IT NL**

(71) Applicant: **Council of Scientific and Industrial Research Rafi Marg New Delhi 110 001(IN)**

(72) Inventor: **Pradhan, Ajit Ramchandra National Chemical Laboratory Pune-411008, Maharashtra(IN)**
Inventor: **Rao, Bollapragad Seshagiri National Chemical Laboratory Pune-411008, Maharashtra(IN)**

(74) Representative: **Collier, Jeremy Austin Grey et al J.A.Kemp & Co., 14 South Square, Gray's Inn London WC1R 5LX (GB)**

(54) An improved process for the production of cumene.

(57) An improved process is disclosed for the preparation of cumene. Cumene is prepared by reacting benzene with a propylating agent in the presence of a catalyst containing metal loaded Zeolite EU-1 in a reactor in the range of a temperature of 150 to 250°C and a pressure of 1 to 35 atmospheres, the propyl and diisopropylbenzene so formed are separated from the reactor effluent by conventional methods. The diisopropylbenzene is recycled back to the reactor. Simultaneous alkylation and transalkylation reactions occur in a single catalyst bed containing Zeolite EU-1 with a feed containing benzene, propylene and diisopropylbenzene. Cumenes are important chemical precursors in the production of detergents and polymers among others.

This invention relates to an improved process for the preparation of cumene by the propylation of benzene with propylating agents in the presence of zeolite catalysts. More specifically, the present invention relates to a process for propylation of benzene to isopropylbenzene in the presence of zeolite EU-1.

The alkylation of aromatics with alkylating agents in the presence of an alkylation catalyst is well known in the prior art to produce such monoalkylated products as ethylbenzene, isopropylbenzene or cumene and linear alkylated benzenes. Such monoalkylated products are important chemical precursors in the production of detergents and polymers among others. Alkylation catalysts that are known to produce alkyl aromatic compounds include the Friedel-Craft catalyst, sulphuric acid, phosphoric acid, $AlCl_3$, clays, zeolites and amorphous silica-alumina.

The propylation of benzene to cumene using propylene as the alkylating agent with solid phosphoric catalyst is in commercial practice for more than two decades. U.S. patent 4,774,377 describes an alkylation-transalkylation process for the production of cumene using solid phosphoric acid catalyst as the alkylation catalyst and zeolite Mordenite as a transalkylation catalyst. U.S. patent 4,395,372 describes an alkylation process using rare earth exchanged Faujasite type zeolite as alkylation catalyst in the presence of sulphur dioxide. U.S. Patent 4,469,908 describes a process for alkylation of aromatic hydrocarbons using ZSM-12.

Even though a variety of catalysts have been claimed to be useful in the propylation of benzene to cumene using propylene as an alkylating agent, the only material to be used in the commercial practice is solid phosphoric acid catalyst. The use of isopropyl alcohol as an alkylating agent is not in commercial practice.

While catalysts based on solid phosphoric acid are universally used in the process for cumene production, there are number of limitations in their use in the mentioned process.

(1) they are extremely ensitive to the moisture content in the propylene. The water content has to be controlled precisely in the range of 200-500 ppm.

(2) a second limitation of the prior art phosphoric acid catalyst is that the phosphoric acid leached out during the process even while operating within the limits of water content specified above, leads to the corrosion of downstream equipments.

(3) a third limitation is that an acid slug containing phosphoric acid has to be periodically disposed off leading to an environmental pollution.

(4) a fourth limitation of the solid phosphoric acid catalyst is that it cannot be regenerated leading to total catalyst life of about one year only.

(5) a fifth limitation of a solid phosphoric acid catalyst is that it cannot transalkylate the diisopropyl benzene to cumene.

While zeolite catalyst based on Faujasite, Mordenite, ZSM-12 and ZSM-5 have been claimed to be useful in the propylation of benzene to cumene, no zeolite based propylation process is in the commercial practice today. The limitations are (1) fast deactivation of above mentioned zeolites due to coke formation, leading to low cycle length of less than six months, (2) the high concentration of the diisopropyl benzenes in the products, (3) low tolerance of zeolites like Faujasite and Mordenite to even low levels of water usually present in the propylene feedstock.

It is hence desirable to develop an improved process utilising catalysts which donot have the limitations of the above mentioned solid phosphoric catalyst or zeolite Faujasite, Mordenite, ZSM-12 and ZSM-5.

The present invention provides an improved process for the preparation of cumene which comprises reacting benzene with propylating agent in the presence of catalyst containing metal loaded zeolite EU-1 in a reactor at a temperature in the range of 150°C to 250°C and a pressure of 1 to 35 atmospheres separating the propyl and diisopropyl benzenes from the reactant effluent by conventional methods and recycling diisopropyl benzene back to the said reactor.

Zeolite Eu-1 is a novel medium-pore zeolite whose preparation was first claimed in 1981 in European Patent No. 42226. Its framework structure has been elucidated only recently (Zeolites Vol. 8, January 1988). Zeolite Eu-1 is a high silica zeolite having 10-membered ring pore system having unidimentional channels in the direction of 10-membered windows. However the characteristics of the zeolite Eu-1 is the presence of side - pockets of these channels. The presence of sidepockets makes this zeolite unique of its kind. The various factors that influence the preparation of zeolite Eu-1 are well studied. Hence the preparation and structure of zeolite Eu-1 doesnot form the subject of the present invention. The present invention describes the application and superiority of the zeolite Eu-1 over conventional catalysts in the propylation of benzene to cumene in high yield.

In one embodiment of the present invention the metal loaded zeolite EU-1 used as a catalyst in the propylation of benzene to cumene has a silica to alumina molar ratio in the framework above 20.

In another embodiment of the present invention, the propylating agent is either propylene or propyl alcohol. It has been found that unlike prior art catalyst including other zeolites like faujasite, mordenite and

EP 0 537 389 A1

ZSM-5, Zeolite Eu-1 retains its activity and selectivity for the propylation reaction even in the presence of substantial quantity of water.

In another embodiment of the present invention the diisopropylbenzenes in the reactor effluent are separated and recycled back to the alkylation reactor where they undergo transalkylation with benzene to yield cumene. The selectivity for the conversion of propylene to cumene in the present invention for example is around 97-98 % compared to 93-95 % in the prior art process using solid phosphoric acid catalyst.

The zeolite EU-1 used as a catalyst in the process of present invention is characterised by X-Ray diffraction pattern given in table 1.

Table 1

| X-Ray diffraction pattern of zeolite EU-1 | |
|---|---|
| d (A°) | I/I° |
| 11.0 ± 0.4 | S |
| 10.1 ± 0.3 | S |
| 9.7 ± 0.2 | W |
| 4.64 ± 0.1 | M |
| 4.30 ± 0.1 | VS |
| 3.99 ± 0.1 | S |
| 3.81 ± 0.08 | M |
| 3.68 ± 0.08 | M |
| 3.43 ± 0.07 | W |
| 3.36 ± 0.07 | W |
| 3.28 ± 0.07 | M |
| 3.24 ± 0.07 | W |

Its adsorption potential for some hydrocarbon molecules is illustrated by the data in table 2

## Table 2 : Adsorption data for zeolite EU-1

### Temp. : 298 °K

### $P/P_O$: 0.5

| Solvent | Wt. % |
|---|---|
| n-Hexane | 14.0 |
| Cyclohexane | 2.2 |
| Cumene | 9.5 |

These data were obtained in a conventional gravimetric adsorption apparatus at a relative partial pressure of the adsorbate of 0.5 . It is desirable to incorporate the zeolite EU-1 in another material more resistent to the temperature and other conditions employed in the process. Such matrix material include synthetic or naturally occuring substances such as clay, silica, alumina or metal oxides. The relative proportion of the EU-1 zeolite and matrix binder material on anhydrous basis may vary widely with the zeolite content ranging between 10 to 90 % by weight .

The molar ratio of benzene to propylating agent employed may range between 20/1 to 1/1 more preferably 10/1 to 1/1 . Reaction is suitably accomplished utilising a feed weight hourly space velocity

3

(WHSV) between 1 to 4. The later WHSV is based upon the total weight of active catalyst and binder thereof.

In one embodiment of the process of the present invention metals like sodium, potassium, calcium or barium are advantageously incorporated into zeolite EU-1 to increase the selectivity to cumene. These metals may be incorporated into zeolite EU-1 either during the synthesis or subsequently by ion-exchange after its incorporation with binder. It has also been found out that a mixture of the above mentioned metals also has an advantageous effect in enhancing the selectivity of the catalyst. The total quantity of such added metals may vary between 0.1 to 5.0 % wt.

The following examples will serve to illustrate the process of this invention without limiting the scope or utility thereof. The catalyst of all cases contained zeolite EU-1 prepared as described in European Patent No. 42226.

Example 1 :

This example illustrates the preparation of the catalyst composite material used in the process of the present investigation. 50 gm of zeolite EU-1 prepared as given in European Patent No. 42226 of 1981 and having silica to alumina mole ratio 40 and Na content 0.1% was mixed with 30 gm of alumina hydrate and extruded into cylindrical pellets of 1/16 " diameter. The extrudates were dried and calcined at 450°C in air for 10 hrs. The X-Ray pattern and adsorption properties of zeolite EU-1 are given in table 1 and 2 respectively.

Example 2:

This example describes the process for the production of cumene using the catalyst prepared as described in example 1. 10 gm of the catalyst as prepared in example 1 is loaded in a fixed bed, down flow, high pressure, high temperature catalytic reactor. Benzene, propylene, propane were passed through the catalytic bed as detailed below. The product analysis is represented in table 3 and compared with that of solid phosphoric catalyst.

Table 3

| Comparison of Zeolite EU-1 with conventional solid phosphoric acid catalyst (SPA): | | |
|---|---|---|
| | SPA | Zeolite |
| MR (Benzene/Propylene) | 8 | 7 |
| WHSV (hr$^{-1}$) | 1.25 | 3.5 |
| Temperature (°C) | 210 | 210 |
| Pressure | atm | atm |
| T.O.S.(hr) | 5 | 5 |
| Nonaromatics | 9.80 | 0.10 |
| Benzene | 77.00 | 78.86 |
| Tol + $C_8A$ | 0.08 | 0.06 |
| Cumene | 11.80 | 19.56 |
| nPB | 0.01 | 0.07 |
| $C_9$ - $C_{11}$ A | - | 0.06 |
| $_\epsilon$DIPB | 1.00 | 1.33 |
| HA | 0.03 | 0.03 |

Example 3:

This example (table 4) indicates the effect of temperature on cumene production. The catalyst of example 1 is used. The catalyst also contains 1.0% Ca, wt.

## Table 4: Effect of Temperature:

$$B/P = 7.3 \; ; \; WHSV = 3.0 \; hr^{-1} \; ; \; Pressure = Atm$$

| Temperature (°C) | 180 | 210 | 240 | 260 |
|---|---|---|---|---|
| Nonaromatics | 0.26 | 0.01 | 0.05 | 0.25 |
| Benzene | 81.74 | 79.23 | 80.98 | 82.50 |
| Tol + $C_8$ A | 0.02 | 0.06 | 0.04 | 0.16 |
| Cumene | 16.38 | 18.22 | 17.44 | 14.95 |
| nPB | 0.01 | 0.19 | 0.22 | 1.05 |
| $C_9$-$C_{11}$ A | 0.01 | 0.07 | 0.08 | 0.31 |
| $\epsilon$DIPB | 1.54 | 1.68 | 1.14 | 0.68 |
| HA | 0.03 | 0.03 | 0.01 | 0.09 |
| Sel. Cum. % | 89.7 | 87.7 | 91.7 | 85.4 |

Example 4:

The influence of benzene to propylene mole ratio on cumene formation is studied and reported in this example (table 5).

5

## Table 5: Effect of mole ratio of Benzene to Alkylating agent

### Temperature: 210°C ; Pressure: Atm; WHSV: 3.0 hr$^{-1}$

| MR | 6.8 | 8.2 | 12.7 | 24 |
|---|---|---|---|---|
| Nonaromatics | 0.03 | 0.03 | 0.02 | 0.01 |
| Benzene | 78.66 | 81.93 | 88.21 | 93.59 |
| Tol + $C_8A$ | 0.04 | 0.03 | 0.02 | 0.02 |
| Cumene | 18.82 | 16.42 | 11.06 | 6.18 |
| nPB | 0.02 | 0.02 | 0.01 | 0.04 |
| $C_9-C_{11}A$ | 0.04 | 0.03 | 0.01 | 0.01 |
| $\epsilon$DIPB | 2.36 | 1.53 | 0.64 | 0.15 |
| HA | 0.03 | 0.02 | 0.01 | - |
| Sel. Cum. % | 88.2 | 90.9 | 93.8 | 96.4 |

Example 5:

The influence of weight hourly space velocity on selectivity of cumene is presented in the example (table 6).

## Table 6: Effect of Space Velocity

M/R = 6.8 ; Temperature = 210°C ; pressure = Atm

| WHSV (hr$^{-1}$) | 1.5 | 3.0 | 5.5 | 11.0 |
|---|---|---|---|---|
| Nonaromatics | 0.03 | 0.02 | 0.01 | 0.08 |
| Benzene | 79.59 | 78.66 | 78.68 | 79.17 |
| Tol. + $C_8A$ | 0.02 | 0.10 | 0.03 | 0.02 |
| Cumene | 18.28 | 18.82 | 18.82 | 18.14 |
| nPB | 0.03 | 0.02 | 0.02 | 0.03 |
| $C_9-C_{11}A$ | 0.03 | 0.04 | 0.01 | 0.03 |
| $\epsilon$DIPB | 1.97 | 2.36 | 2.38 | 2.48 |
| HA | 0.04 | 0.03 | 0.03 | 0.03 |
| Sel. Cum. % | 89.6 | 88.2 | 88.3 | 87.1 |

Example 6:

The various cumene contents in the products obtained at different pressures is illustrated in this example (table 7).

7

## Table 7: Effect of Pressure

### Temperature: 210°C ; WHSV: 3 hr$^{-1}$ ; MR: 7

| Pressure | Atm | 25 Kg/cm$^2$ |
|---|---|---|
| Nonaromatics | 0.10 | 0.02 |
| Benzene | 78.86 | 78.92 |
| Tol. + $C_8A$ | 0.06 | 0.03 |
| Cumene | 19.56 | 19.20 |
| nPB | 0.07 | 0.05 |
| $C_9$–$C_{11}A$ | 0.06 | 0.02 |
| $\epsilon$DIPB | 1.33 | 1.76 |
| HA | 0.03 | – |
| Sel. Cum. % | 92.6 | 91.1 |

Example 7:

This example (table 8) includes the results of the catalyst aging characteristics with time on stream at atmospheric pressure. The catalyst contained 0.2% Na and 2.1% Ba, wt.

## Table 8: The catalyst aging characteristics

### Temperature: 210°C ; Pressure: Atm ; WHSV: 3hr ; MR: 7

| TDS (hrs) | 22 | 80 | 178 | 280 |
|---|---|---|---|---|
| Nonaromatics | 0.10 | 0.10 | 0.06 | 0.13 |
| Benzene | 78.73 | 78.86 | 79.32 | 79.93 |
| Tol. + $C_8A$ | 0.06 | 0.06 | 0.01 | – |
| Cumene | 19.43 | 19.50 | 18.66 | 18.03 |
| nPB | 0.35 | 0.07 | 0.01 | 0.01 |
| $C_9-C_{11}A$ | 0.11 | 0.06 | 0.04 | 0.02 |
| εDIPB | 1.09 | 1.33 | 1.86 | 1.77 |
| HA | 0.10 | 0.03 | 0.03 | 0.09 |
| Sel. Cum. % | 90.1 | 92.2 | 90.2 | 89.8 |

Example 8:

This example (table 9) illustrate the regenerability of the catalyst. The deactivated catalyst was regenarated and tested for its activity.

## Table 9: Catalyst Regenerability

### Temperature:210°C ; WHSV: 3 $hr^{-1}$ ; Pressure: Atm ; MR: 7

|  | Fresh | After Regeneration |
|---|---|---|
| Nonaromatics | 0.12 | 0.12 |
| Benzene | 79.85 | 79.63 |
| Tol. + $C_8A$ | 0.08 | 0.08 |
| Cumene | 18.32 | 18.22 |
| nPB | 0.45 | 0.19 |
| $C_9-C_{1}1A$ | 0.12 | 0.07 |
| $\epsilon$DIPB   1.33 | 1.00 | 1.28 |
| HA       0.03 | 0.04 | 0.03 |
| Sel. Cum. % | 90.9 | 89.5 |

Example 9:

The example (table 10) illustrates the simultaneous occurance of both alkylation and transalkylation reactions in a single catalyst bed containing zeolite EU-1 with feed containing benzene, propylene and diisopropylbenzenes. The silica to alumina ratio of the catalyst was 40. The catalyst contained 0.5% Na, 0.8% Ca and 0.1% K, wt.

## Table 10: Simultaneous Alkylation and Transalkylation

### Temperature: 210°C ; WHSV: 3 hr$^{-1}$ ; MR: 8 ;

### The feed contains 3% DIPB

|                     | Product |
|---------------------|---------|
| Nonaromatics        | 0.05    |
| Benzene             | 77.01   |
| Tol. + $C_8A$       | 0.04    |
| Cumene              | 19.45   |
| nPB                 | 0.03    |
| $C_9$-$C_{11}A$     | 0.01    |
| $\epsilon$DIPB      | 3.29    |
| HA                  | 0.01    |
| Sel. Cum. %         | 97.2    |

Example 10:

This example (table 11) shows the use of isopropanol as an alkylating agent in the place of propylene.

## Table 11: Effect of Alkylating Agent

### Temperature: 220°C ; WHSV: 3.5 hr$^{-1}$ ; Pressure: Atm

| Alkylating Agent | Propylene | Isopropanol |
|---|---|---|
| Nonaromatics | 0.17 | 0.03 |
| Benzene | 77.14 | 78.66 |
| Tol. + $C_8A$ | 0.16 | 0.04 |
| Cumene | 19.40 | 18.82 |
| nPB | 0.02 | 0.02 |
| $C_9$-$C_{11}A$ | 0.06 | 0.04 |
| DIPB | 2.94 | 2.36 |
| HA | 0.11 | 0.03 |
| Sel. Cum. % | 84.9 | 88.2 |

Example: 11

This example (table 12) includes the results on transalkylation reaction of a feed containing benzene and diisopropyl benzene. The catalyst contained 0.5% Na, 0.5% Ca and 0.2% Ba.

## Table 12: Transalkylation Reaction

Temperature: 210°C ; Pressure: Atm ; MR: 20 ; WHSV: 3 $hr^{-1}$

|  | Product |
|---|---|
| Nonaromatics | 0.12 |
| Benzene | 89.65 |
| Tol. + $C_8A$ | 0.04 |
| Cumene | 5.42 |
| nPB | 0.06 |
| $C_9-C_{11}A$ | 0.06 |
| $\epsilon$DIPB | 4.71 |
| HA | 0.02 |

## Claims

1. An improved process for the preparation of cumene which comprises reacting benzene with a propylating agent in the presence of a catalyst containing metal loaded zeolite EU-1 in a reactor at a temperature in the range of 150 to 250°C and pressure in the range of 1 to 35 atmospheres, separating the propyl and diisopropylbenzenes from the reactor effluent by conventional methods and recycling the diisopropylbenzene back to the said reactor.

2. The process as claimed in claim 1 wherein the propylating agent is selected from propylene and propyl alcohol.

3. The process as claimed in claim 1 wherein the molar ratio of benzene and propylating agent in the mixture is between 1 and 20, preferably between 1 and 10.

4. The process as claimed in claim 1 wherein the reaction is effected at a weight hourly space velocity (WHSV) (benzene plus propylating agent) of 1 to 4.

5. The process as claimed in claim 1 wherein the metal loaded zeolite EU-1 has a silica to alumina ratio above 20.

6. The process as claimed in claim 1 wherein the metal in the metal loaded zeolite EU-1 is loaded with one or more of the metals selected from the group sodium, calcium, potassium and barium.

7. An improved process as claimed in claim 1 wherein the amount of metal loaded into zeolite EU-1 ranges from 0.1 to 0.5% by weight.

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,A | EP-A-0 042 226 (ICI)<br>* page 5, line 10 - page 11, line 32; claims * | 1-7 | C07C2/66<br>C07C2/86<br>C07C6/12<br>C07C15/085 |
| | --- | | |
| A | EP-A-0 366 515 (INSTITUT FRANCAIS DU PETROLE)<br>* claims; examples * | 1-7 | |
| | ----- | | |

TECHNICAL FIELDS SEARCHED (Int. Cl.5 )

C07C

The present search report has been drawn up for all claims

| Place of search | Date of completion of the search | Examiner |
|---|---|---|
| THE HAGUE | 24 JUNE 1992 | ZERVAS B. |

EPO FORM 1503 03.82 (P0401)